# EUROPÄISCHE PATENTANMELDUNG

(11) **EP 4 512 347 A1**
(43) Veröffentlichungstag der Anmeldung: **26.02.2025**
(21) Anmeldenummer: 24182525.6
(22) Anmeldetag: 17.06.2024
(51) Int. Cl.: A61B 10/00, G01N 33/543

(54) **VORRICHTUNG UND VERFAHREN ZUM VISUELLEN NACHWEIS VON ANALYTEN IN EINER SPEICHELPROBE**

(30) Priorität: 12.07.2023 DE 102023118482
(71) Anmelder: Protzek Biotec GmbH, 4415 Lausen (CH)
(72) Erfinder: Protzek, Christoph, 79539 Lörrach (DE)
(74) Vertreter: Patentanwälte Dörner & Kötter PartG mbB

(57) **Zusammenfassung**

Die Erfindung betrifft eine Vorrichtung zum visuellen Nachweis von Analyten in einer vom menschlichen oder tierischen Organismus stammenden Speichelprobe, umfassend eine Trägerplatte (1), die aus Kartonage hergestellt ist und auf der wenigstens ein mit einem Probeaufnahmeabschnitt (31) und einem Testabschnitt (32) versehener Teststreifen (3) angeordnet ist, wobei der wenigstens eine Teststreifen (3) über eine Deckplatte (2) zumindest bereichsweise überdeckt ist, wobei die Trägerplatte (1) dem Teststreifen (3) vorgelagert einen mit einer Speichelsammelrippe (14) versehenen Speichelsammelabschnitt (13) aufweist, und wobei die Deckplatte (2) einen Freigabeabschnitt (24) aufweist, der den Speichelsammelabschnitt (13) zumindest bereichsweise überdeckt und der von diesem entfernbar ist. Die Erfindung betrifft weiterhin ein Verfahren zur Analyse einer Speichelprobe mit einer solchen Vorrichtung.

## Beschreibung

Die Erfindung betrifft eine Vorrichtung zum visuellen Nachweis von Analyten in einer vom menschlichen oder tierischen Organismus stammenden Speichelprobe nach dem Oberbegriff des Patentanspruchs 1. Die Erfindung betrifft weiterhin ein Verfahren zum visuellen Nachweis von Analyten in einer Speichelprobe nach dem Patentanspruch 11.

Vorrichtungen zum Nachweis von Analyten in einer flüssigen Probe, zum Beispiel von Drogen, Medikamenten oder Antikörpern als Zeichen für bestimmte Erkrankungen in einer Körperflüssigkeit oder Feststellungen im Sinne von Konzentrationsangaben sind in unterschiedlichen Ausführungen bekannt. Besonderes Interesse besteht an solchen Geräten, die auch außerhalb eines Labors eingesetzt werden können. Diese müssen tragbar und leicht zu bedienen sein sowie in kurzer Zeit ein zuverlässiges Ergebnis liefern. Insbesondere zur Durchführung von Tests auf Drogenkonsum oder Medikamentenmissbrauch oder dergleichen durch die Polizei ist eine Vorrichtung erforderlich, die auch im Freien bei unterschiedlichen wechselnden Lichtverhältnissen, Wetterverhältnissen und Temperaturen einen derartigen Test ermöglicht.

In der DE 20 2014 002 369 U1 wird hierzu ein Gerät vorgeschlagen, in das eine Testkassette einführbar ist und das mit einer Lichtquelle versehen ist, mittels derer die untere Wand der aus einem lichtdurchlässigen Kunststoff bestehenden Testkassette sowie den Testbereich des in der Testkassette angeordneten Teststreifens durchstrahlt und das Testergebnis ausleuchtet. Die Testkassette ist zur Durchführung eines lateral flow immuno-assays, Aptamer basierter Assay oder Encym immuno assay (EIA) ausgelegt und besteht aus einem unteren Deckel und einem oberen Deckel. Eingeschlossen von diesen beiden Deckeln enthält die Testkassette einen Teststreifen, der innerhalb einer Aufnahme in Position gehalten wird. In dem oberen Deckel befindet sich über dem Probenaufnehmer des Teststreifens eine Einfüllöffnung, durch die eine zu untersuchende flüssige Probe eingegeben werden kann. Über den mittleren Testbereich des Teststreifens befindet sich ein Kontrollfenster, durch welches das Testergebnis abgelesen werden kann. Derartige Kassetten haben sich im mobilen Einsatz bewährt. Nachteilig an den vorbekannten Kassetten ist jedoch, dass dessen Herstellung aufwendig ist und diese relativ viel Platz benötigen.

Zur Lösung dieser Problematik wird in der DE 20 2016 104 645 U1 hierzu eine Vorrichtung vorgeschlagen, die eine im Wesentlichen aus Pappe hergestellte Trägerschicht umfasst, die mit einer Deckfolie versehen ist und bei der Teststreifenaufnahmen und Reservoire durch Einprägungen oder durch Ausnehmungen einer zwischen Deckfolie und Trägerplatte angeordneten Zwischenschicht ausgebildet sind. Diese Vorrichtung zeichnet sich besonders dadurch aus, dass sie sehr leicht und platzsparend, günstig herstellbar und darüber hinaus umweltfreundlich zu entsorgen ist.

Weitere Vorrichtungen zum visuellen Nachweis von Analyten in einer vom menschlichen oder tierischen Organismus stammenden Flüssigkeitsprobe sind in der EP 3 287 785 A1, DE 20 2017 100 656 U1, US 2003/0 175 992 A1 und in der AU 2018271367 A1 offenbart.

Voraussetzung für die Funktion der bei den vorgenannten Vorrichtungen zum Einsatz kommenden Teststreifen ist die Aufgabe einer fließfähigen Probenflüssigkeit in ausreichender Menge.

Zum qualitativen Nachweis von Drogen im menschlichen Speichel sind Speicheltests in unterschiedlichen Ausführungen bekannt. Dabei wird der Speichel zunächst dem Probanden mittels eines Schwamms aus der Mundhöhle entnommen und nachfolgend einem immunochromatographischen Teststreifen zugeführt ("Lateral Flow" - Technologie). Da Drogenkonsumenten aufgrund der physiologischen Wirkung der Drogen häufig einen sehr trockenen Mund haben, steht nur wenig Speichel für die Probennahme zur Verfügung. Zur Durchführung des Tests wird dieser Speichel einer Pufferflüssigkeit zugegeben, welche sodann auf den Teststreifen aufgegeben wird. Nachteilig an diesem Verfahren ist, dass durch die Verdünnung der Speichelprobe eine Senkung der Konzentration des Analyten in der Probe erfolgt, wodurch der eigentliche Test beeinträchtigt ist. Andere Testverfahren erweisen sich als komplex und aufwändig bedienbar, wodurch sie für den mobilen Einsatz beispielsweise bei der Verkehrspolizei ungeeignet erscheinen.

Hier will die Erfindung Abhilfe schaffen. Der Erfindung liegt die Aufgabe zugrunde, eine Vorrichtung zum visuellen und messtechnischen Nachweis von Analyten in einer Speichelprobe bereitzustellen, die einfach aufgebaut ist, für den mobilen Einsatz geeignet ist und die auch bei Vorliegen einer geringen Probenmenge ein unverfälschtes Testergebnis ermöglicht. Gemäß der Erfindung wird diese Aufgabe durch eine Vorrichtung mit den Merkmalen des Patentanspruchs 1 gelöst.

Mit der Erfindung ist eine Vorrichtung zum visuellen und messtechnischen Nachweis von Analyten in einer Speichelprobe bereitgestellt, die einfach aufgebaut ist, für den mobilen Einsatz geeignet ist und die auch bei Vorliegen einer geringen Probenmenge ein unverfälschtes Testergebnis ermöglicht. Dadurch, dass die Trägerplatte 1 dem Teststreifen vorgelagert einen mit einer Speichelsammelrippe versehenen Speichelsammelabschnitt aufweist, wobei die Deckplatte einen Freigabeabschnitt aufweist, der den Speichelsammelabschnitt zumindest bereichsweise überdeckt und von diesem entfernbar ist, ist durch Überstreichen einer Zunge mit der Speichelsammelrippe des Speichelsammelabschnitt die Ausbringung einer maximalen Speichelprobenmenge auf den Teststreifen ermöglicht. Unter dem Begriff "Teststreifen" ist vorliegend ein immunochromatographischer Teststreifen (Lateral Flow Teststreifen) zu verstehen.

In Weiterbildung der Erfindung überragt der Freigabeabschnitt die Trägerplatte bereichsweise. Hierdurch ist der Freigabeabschnitt einfach zu greifen, um ihn von der Trägerplatte zu entfernen,

In Ausgestaltung der Erfindung ist die Speichelsammelrippe im Wesentlichen U-förmig ausgebildet. Hierdurch ist ein maximales Sammeln von Speichel durch Überstreichen einer Zunge ermöglicht.

In weiterer Ausgestaltung der Erfindung ist in der Trägerplatte eine Sammelmulde eingebracht, in welcher der Probeaufnahmeabschnitt zumindest eines Teststreifens zumindest bereichsweise angeordnet ist. Hierdurch ist eine gebündelte Aufgabe des gesammelten Speichels auf den Probeaufgabenabschnitt des Teststreifens erzielt. Bevorzugt ragt die Sammelmulde bereichsweise in den Speichelsammelabschnitt hinein.

In weiterer Ausgestaltung der Erfindung weist die Speichelsammelrippe trichterartig zulaufende Leitrippenabschnitte auf, die an die Sammelmulde angrenzen. Hierdurch ist eine gerichtete Zuführung des gesammelten Speichels zu der Sammelmulde erzielt.

In weiterer Ausgestaltung der Erfindung sind die Trägerplatte und die Deckplatte aus Pappe hergestellt. Hierdurch ist eine kostengünstige Herstellung einer umweltverträglichen Vorrichtung ermöglicht.

In Weiterbildung der Erfindung ist die Speichelsammelrippe in die Trägerplatte eingeprägt. Hierdurch ist eine einfache und kostengünstige Realisierung einer in die Trägerrippe integrierten Sammelrippe ermöglicht.

In Ausgestaltung der Erfindung ist die Deckplatte über eine Knick- oder Perforationslinie mit dem Freigabeabschnitt verbunden, über die dieser nach außen abknickbar ist. Hierdurch ist ein einfaches Verschwenken des Freigabeabschnitts um nahezu 180° ermöglicht, sodass dieser beim Überfahren einer Zunge mit dem so freigegebenen Speichelsammelabschnitt nicht im Wege ist.

In weiterer Ausgestaltung der Erfindung ist in der Deckplatte ein Trägerflüssigkeitsaufgabefenster eingebracht, das im äußeren Bereich des Probeaufnahmeabschnitts des Teststreifens oder in der Sammelmulde mündet. Hierdurch ist eine Zuführung von Trägerflüssigkeit hinter der Speichelprobe zu deren "Transport" durch den Teststreifen ermöglicht.

In Weiterbildung der Erfindung ist das Probeaufgabefenster über eine zumindest bereichsweise abziehbare Folie verschlossen. Hierdurch ist einer Kontamination der Teststreifen vor deren Benutzung verhindert.

Der vorliegenden Erfindung liegt die weiterhin die Aufgabe zugrunde, ein Verfahren zum visuellen und messtechnischen Nachweis von Analyten in einer Speichelprobe bereitzustellen, das für den mobilen Einsatz geeignet ist und das auch bei Vorliegen einer geringen Probenmenge ein unverfälschtes Testergebnis ermöglicht. Gemäß der Erfindung wird diese Aufgabe durch ein Verfahren mit den Merkmalen des Patentanspruchs 12 gelöst. Dadurch, dass zunächst der Freigabeabschnitt der Deckplatte umgeknickt wird und nachfolgend der freigelegte Speichelsammelabschnitt der Trägerplatte mit seiner Speichelsammelrippe über die Zunge eines Probanden bewegt wird, ist die Sammlung einer maximalen Speichelmenge ermöglicht.

In Weiterbildung der Erfindung wird eine Menge an Trägerflüssigkeit über ein in der Deckplatte angeordnetes Trägerflüssigkeitsaufgabefenster hinter die Speichelprobe auf den Probenaufnahmeabschnitt (31) des Teststreifens (3) gegeben. Hierdurch ist ein "Transport" der Speichelprobe durch den Teststreifen erzielt.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:
- Figur 1: die schematische Darstellung eines Kartonagenzuschnitts einer Vorrichtung zum visuellen Nachweis von Analyten in einer Speichelprobe mit eingelegtem Teststreifen,
- Figur 2: die schematische Darstellung der Speichelprobelsammlung mit einer aus dem Kartonagenzuschnitt aus Figur 1 hergestellten Vorrichtung mit zurückgeklapptem Freigabeabschnitt und
- Figur 3: die schematische Darstellung der Vorrichtung aus Figur 2 nach der Speichelprobenentnahme in der Auswertungsphase.

Die als Ausführungsbeispiel gewählte Vorrichtung zum visuellen Nachweis von Analyten in einer Speichelprobe besteht im Wesentlichen aus einer Trägerplatte 1, die entlang ihrer Längsseite schwenkbar mit einer Deckplatte 2 verbunden ist. Trägerplatte 1 und Deckplatte 2 sind im Ausführungsbeispiel aus Pappe hergestellt und aus einem einzigen mittig mit einer Knickfalz 11 versehenen Kartonzuschnitt gebildet. Auf der Trägerplatte 1 ist eine Teststreifenaufnahme 12 eingeprägt, in die ein Teststreifen 3 eingelegt ist, der einen Probeaufnahmeabschnitt 31, einen Testabschnitt 32 und einen zwischen diesen Abschnitten angeordneten Antikörperbelagabschnitt 33 aufweist. Die Teststreifenaufnahme 12 geht in eine - nicht dargestellte - Sammelmulde über, in welche der Probeaufnahmeabschnitt 31 des Teststreifens 3 positioniert ist, An die Teststreifenaufnahme 12 schließt sich auf der Trägerplatte 1 ein Speichelsammelabschnitt 13 an. Auf dem Speichelsammelabschnitt 13 ist eine U-förmige Sammelrippe 14 angeordnet, die über zwei trichterartig angeordnete Leitrippenabschnitte 15 mit der Sammelmulde verbunden sind, an die diese angrenzen.

Auf der Deckplatte 2 ist im Bereich des Testabschnitts 32 des Teststreifens 3 ein rechteckig ausgeführtes Fenster 21 eingebracht. Beabstandet zu dem Fenster 21 ist eine bogenförmige Halterippe 22 in die Decklatte eingeprägt, welche auf dem Teststreifen aufliegt, wodurch die Fließgeschwindigkeit einer aufgegebenen Speichelprobe positiv beeinflusst wird.

Beabstandet zu der Halterippe 22 ist eine Perforationslinie 23 in die Deckplatte 2 eingebracht, die einen Freigabeabschnitt 24 begrenzt, der über dem Speichelsammelabschnitt 13 positioniert ist, den er abdeckt. Auf der dem Fenster 21 zugewandten Seite der Perforationslinie 23 ist in die Deckplatte 2 weiterhin ein Trägerflüssigkeitsaufgabefenster 25 eingebracht. Die Deckplatte 2 ist im Bereich bis zur Perforationslinie mit der Trägerplatte 1 verklebt. Der Teststreifen 1 ist so zwischen Trägerplatte 1 und Deckplatte 2 in seiner Position gehalten und durch die Deckplatte 2 abgedeckt.

Vor der Durchführung eines Tests sind auf dem Teststreifen 3 keine Linien 321, ersichtlich. Bei der Durchführung eines Tests wird zunächst der Freigabeabschnitt 24 über die als Scharnier wirkende Perforationslinie 23 zurückgeklappt, bis sie auf der Deckplatte 2 aufliegt. Der so freigegebene Speichelsammelabschnitt 13 wird sodann mit seiner Speichelsammelrippe 14 über die Zunge eines Probanden gezogen, sodass durch die U-förmige Speichelsammelrippe 14 Speichel gesammelt und entlang der Leitrippenabschnitte 15 auf den in der - nicht dargestellten - Sammelmulde befindlichen Probeaufnahmeabschnitt 31 des Teststreifens 3 befördert.

Durch Kapillarwirkung fließt die Probenflüssigkeit in Richtung Testabschnitt 32. Sie kommt dabei zunächst mit dem Antikörperbelagabschnitt 33 in Kontakt, in dem sich mit Goldpartikeln konjugierte Antikörper zum Antigen eines festzustellenden Analyten in standardisierter (dosierter) Menge befinden. Die Antikörper weisen eine für verschiedene Analyten (Drogen) unterschiedliche Bindungsaktivität auf. Die mit Goldpartikeln konjugierten Antikörper werden durch die Probenflüssigkeit mitgenommen, welche den Testabschnitt 32 durchfließt.

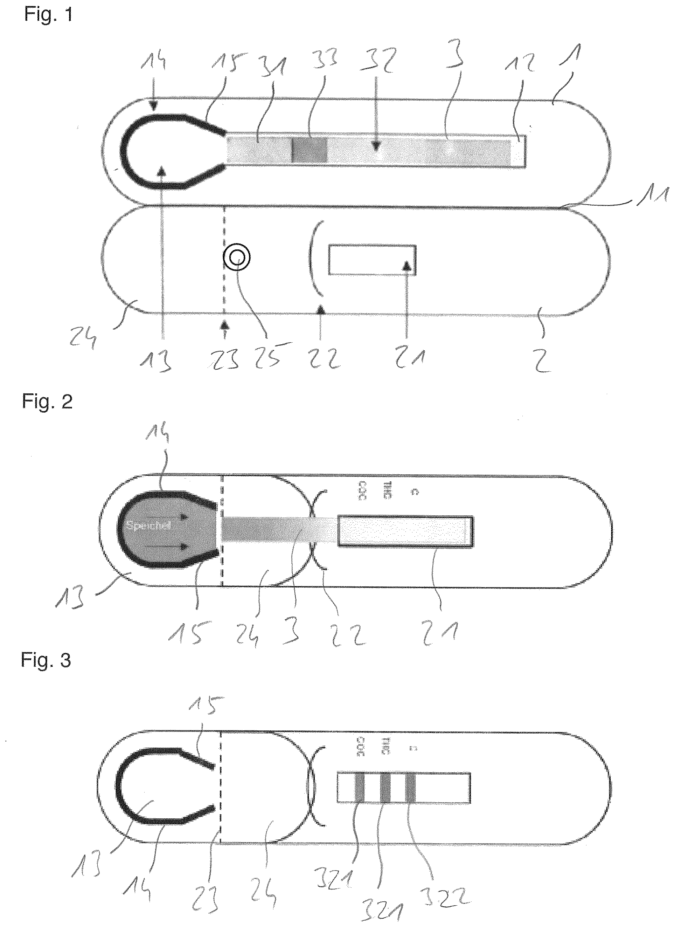

Enthält die Probenflüssigkeit nicht den festzustellenden Analyten, so werden beim Durchfließen des Testsabschnitts 32 die dort im Bereich der Testlinien 321 befindlichen Antigene des Analyten durch die von der Probenflüssigkeit mitgenommenen Antikörper gebunden und es bilden sich sichtbare Testlinien heraus, die durch die mit den Antikörpern konjugierten Goldpartikel eine rote oder braune Farbe erhalten. Enthält die Probenflüssigkeit den festzustellenden Analyten, so werden dessen Antigene bereits durch die Antikörper im Bereich des Antikörperbelagabschnitts 33 des Teststreifens 3 gebunden und es kann im Testabschnitt 32 nicht mehr zu einer Anlagerung kommen, weshalb sich keine sichtbaren Testlinien 321 herausbilden. Die Kontrolllinie 322 zeigt an, ob der Test valide ist. In Figur 3 sind die Testlinien 321 farbig herausgebildet, weshalb der Test in Bezug auf die entsprechenden Analyten negativ ist.

Läuft die Speichelprobe mangels Menge nicht ausreichend in Richtung Testabschnitt 32, so wird in das Trägerflüssigkeitsaufgabefenster 25 auf den darunter befindlichen Probenaufnahmeabschnitt 31 des Teststreifens 3 eine geringe Menge einer Trägerflüssigkeit, beispielsweise ein Tropfen destilliertes Wasser, aufgegeben. Diese Trägerflüssigkeit fließt durch Kapillarwirkung in Richtung Testabschnitt 32 und schiebt dabei die Speichelprobe vor sich her, ohne sich jedoch mit dieser zu vermischen.

Die erfindungsgemäße Vorrichtung zeichnet sich dadurch aus, dass sie bei einer kompakten Ausbildung unter Einsatz geringer Probemengen, insbesondere Speichelprobemengen die Durchführung eines validen Tests ermöglicht. Durch den mit einer Speichelsammelrippe versehenen Speichelsammelabschnitt ist eine vergrößerte Menge an Speichelprobe erzielbar. Durch die Zugabe einer Trägerflüssigkeit, die hinter die Speichelprobe in das Kapillarsystem des Teststreifens 3 gegeben wird, ist ein Transport einer geringen Probemenge durch den Teststreifen 3 ermöglicht, ohne dass eine Vermischung der Trägerflüssigkeit mit der Probe erfolgt.

Durch die Herstellung der Vorrichtung im Wesentlichen aus Kartonwerkstoffen ist zudem eine umweltgerechte Entsorgung dieser zur einmaligen Verwendung bestimmten Vorrichtung ermöglicht.

## Patentansprüche

1. Vorrichtung zum visuellen Nachweis von Analyten in einer vom menschlichen oder tierischen Organismus stammenden Speichelprobe, umfassend eine Trägerplatte (1), die aus Kartonage hergestellt ist und auf der wenigstens ein mit einem Probeaufnahmeabschnitt (31) und einem Testabschnitt (32) versehener Teststreifen (3) angeordnet ist, wobei der wenigstens eine Teststreifen (3) über eine Deckplatte (2) zumindest bereichsweise überdeckt ist, **dadurch gekennzeichnet, dass** die Trägerplatte (1) dem Teststreifen (3) vorgelagert einen mit einer Speichelsammelrippe (14) versehenen Speichelsammelabschnitt (13) aufweist, wobei die Deckplatte (2) einen Freigabeabschnitt (24) aufweist, der den Speichelsammelabschnitt (13) zumindest bereichsweise überdeckt und der von diesem entfernbar ist.

2. Vorrichtung nach Anspruch 1, **dadurch gekennzeichnet, dass** der Freigabeabschnitt (24) die Trägerplatte (1) bereichsweise überragt.

3. Vorrichtung nach Anspruch 1 oder 2, **dadurch gekennzeichnet, dass** die Speichelsammelrippe (14) im Wesentlichen U-förmig ausgebildet ist.

4. Vorrichtung nach einem der vorgenannten Ansprüche, **dadurch gekennzeichnet, dass** in der Trägerplatte (1) eine Sammelmulde eingebracht ist, in welcher der Probeaufnahmeabschnitt (31) zumindest eines Teststreifens (3) zumindest bereichsweise angeordnet ist.

5. Vorrichtung nach Anspruch 4, **dadurch gekennzeichnet, dass** die Sammelmulde bereichsweise in den Speichelsammelabschnitt (13) hineinragt.

6. Vorrichtung nach Anspruch 4 oder 5, **dadurch gekennzeichnet, dass** die Speichelsammelrippe (14) trichterartig zulaufende Leitrippenabschnitte (15) aufweist, die an die Sammelmulde angrenzen.

7. Vorrichtung nach einem der vorgenannten Ansprüche, **dadurch gekennzeichnet, dass** die Trägerplatte (1) und die Deckplatte (2) aus Pappe hergestellt sind.

8. Vorrichtung nach Anspruch 7, **dadurch gekennzeichnet, dass** die Speichelsammelrippe (14) in die Trägerplatte (1) eingeprägt ist.

9. Vorrichtung nach einem der vorgenannten Ansprüche, **dadurch gekennzeichnet, dass** die Deckplatte (2) über eine Knick- oder Perforationslinie (23) mit dem Freigabeabschnitt (24) verbunden ist, über die dieser nach außen abknickbar ist.

10. Vorrichtung nach einem der vorgenannten Ansprüche, **dadurch gekennzeichnet, dass** in der Deckplatte (2) ein Trägerflüssigkeitsaufgabefenster (25) eingebracht ist, das im äußeren Bereich des Probeaufnahmeabschnitts (31) des Teststreifens (3) oder in der Sammelmulde mündet.

11. Vorrichtung nach Anspruch 10, **dadurch gekennzeichnet, dass** das Trägerflüssigkeitsaufgabefenster (25) über eine zumindest bereichsweise abziehbare Folie verschlossen ist.

12. Verfahren zur Analyse einer Speichelprobe mit eine Vorrichtung nach einem der vorgenannten Ansprüche, wobei zunächst der Freigabeabschnitt (24) der Deckplatte (2) umgeknickt wird und nachfolgend der freigelegte Speichelsammelabschnitt (13) der Trägerplatte (1) mit seiner Speichelsammelrippe (14) über die Zunge eines Probanden bewegt wird.

13. Verfahren nach Anspruch 12, **dadurch gekennzeichnet, dass** eine Menge an Trägerflüssigkeit über ein in der Deckplatte (2) angeordnetes Trägerflüssigkeitsaufgabefenster (25) hinter die Speichelprobe auf den Probenaufnahmeabschnitt (31) des Teststreifens (3) gegeben wird.
